(19) Europäisches Patentamt
European Patent Office
Office européen des brevets

(11) EP 2 510 022 B1

(12) **EUROPEAN PATENT SPECIFICATION**

(45) Date of publication and mention of the grant of the patent:
**15.02.2017 Bulletin 2017/07**

(21) Application number: **10776914.3**

(22) Date of filing: **08.11.2010**

(51) Int Cl.:
***C08C 19/24*** *(2006.01)*

(86) International application number:
**PCT/US2010/055834**

(87) International publication number:
**WO 2011/071631 (16.06.2011 Gazette 2011/24)**

(54) **MANUFACTURE OF OLIGOMERS FROM NONENE**

HERSTELLUNG VON OLIGOMEREN AUS NONEN

FABRICATION D'OLIGOMÈRES À PARTIR DE NONÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2009 US 267189 P**

(43) Date of publication of application:
**17.10.2012 Bulletin 2012/42**

(73) Proprietor: **ExxonMobil Chemical Patents Inc.**
**Baytown TX 77520-2101 (US)**

(72) Inventors:
- **YANG, Norman**
**Westfield, NJ 07090 (US)**
- **MATSUNAGA, Philip, T.**
**Houston, TX 77059 (US)**
- **GOZE, Maria, C., B.**
**East Brunswick, NJ 08816 (US)**

(74) Representative: **ExxonMobil Chemical Europe Inc.**
**IP Law Europe**
**Hermeslaan 2**
**1831 Machelen (BE)**

(56) References cited:
**WO-A1-03/020856**
**WO-A1-2007/011973**
**WO-A2-2007/040811**
**US-B1- 6 355 603**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

This invention relates to the manufacture of oligomers. More particularly, this invention relates to certain processes for the manufacture of various poly alpha-olefins prepared from either a single feed of nonene or a mixed feed of olefins comprising nonene, and the poly alpha-olefins produced therefrom.

### BACKGROUND OF THE INVENTION

Poly alpha-olefins comprise one class of hydrocarbon lubricants which has achieved importance in the lubricating oil market. These materials are typically produced by the oligomerization or polymerization of alpha-olefins typically ranging from 1-octene to 1-dodecene, with 1-decene being a preferred material, although polymers of lower olefins such as ethylene and propylene may also be used, including copolymers of ethylene with higher olefins. The poly alpha-olefin (PAO) products may be obtained with a wide range of viscosities varying from highly mobile fluids of about 2cSt at 100° C to higher molecular weight, viscous materials which have viscosities exceeding 100 cSt at 100° C. The PAO's may be produced by the oligomerization or polymerization of olefin feed in the presence of a catalyst such as $AlCl_3$, $AlBr_3$, $BF_3$, or $BF_3$ complexes. Subsequent to the oligomerization or polymerization, the lubricant range products may be hydrogenated in order to reduce the residual unsaturation. In the course of this reaction, the amount of unsaturation is generally reduced by greater than 90%. WO 2003/020856 and WO 2007/011973 relate to poly alpha-olefin oligomers and methods of making such oligomers. US 6,355,603 relates to the thermal reaction products of maleic anhydride and oligoalkenes obtained by metallocene-catalyzed oligomerization of 1-decene.

The automotive industry is placing greater demands on engine oils - operating at higher temperatures for longer times and improving fuel economy; driving a demand for low viscosity PAO's, preferably 4 cSt, while desiring a low Noack volatility and low temperature performance properties. Thus, a need exists for low viscosity PAO's which exhibit low Noack volatility (ASTM D 5800 Standard Test Method for Evaporation Loss of Lubricating Oils by the Noack Method).

The properties of a particular grade of PAO are greatly dependent on the alpha-olefin used to make that product. In general, the higher the carbon number of the alpha-olefin, the lower the Noack volatility and the higher the viscosity index and pour point of the product. Conversely, the lower the carbon number of the alpha-olefin, the higher the Noack volatility and the lower the viscosity index and pour point of the product. For either olefin used, for automotive applications, the desired low Noack volatility and the lower pour point are generally conflicting goals for a PAO.

It would be desirable to manufacture poly alpha-olefins using a wider variety of alpha-olefins such that an optimal balance of volatility and low temperature properties could be more easily achieved. Currently, with available basestocks, to achieve a balance of desired properties in the finished PAO, after oligomerization or polymerization and possible hydrogenation, multiple distillation cuts and mixing of the different cuts is utilized. This adds to the manufacturing time and effort and the present invention is directed to new basestocks and basestock blends to provide for improved PAOs.

### SUMMARY OF THE INVENTION

Disclosed herein is a process for the oligomerization or polymerization of alpha-olefins. The feedstock comprises a blend of alpha-olefins comprising more than 10 wt% nonene and at least one alpha-olefin selected from the group consisting of decene, dodecane, and tetradecene. The nonene comprising alpha-olefin feedstock and at least one oligomerization or polymerization catalyst system are subjected to oligomerization or polymerization conditions in a reactor to oligomerized or polymerize the alpha-olefins. Following reaction, the mixture may be subjected to distillations to remove unreacted alpha-olefins and dimers of the alpha-olefins. The resulting product may also be hydrogenated. The final product may also be fractioned to recover at least two fractions of poly alpha-olefins of differing nominal viscosities.

In embodiments wherein the feedstock is a blend of nonene and at least two other alpha-olefins, the feedstock contains more than 10 to 80 wt% nonene.

In the various embodiments and different feedstocks, the catalyst system used for oligomerization or polymerization contains a catalyst selected from the group consisting of a Friedel-Crafts catalyst, a supported reduced metal oxide catalyst, an acidic ionic liquid, a bridged substituted aromatic transition metal compound, and an unbridged substituted aromatic transition metal compound.

In other embodiments, the catalyst is selected from the group consisting of aluminum halides, $BF_3$, a compound represented by the formula (1) (Cp-A'-Cp*)$MX_1X_2$, and a compound represented by the formula (2) (CpCp*)$MX_1X_2$ wherein M is a metal center; Cp and Cp* are the same or different cyclopentadienyl rings that are each bonded to M, and substituted with from zero to four substituent groups for formula (1) and zero to five substituents for formula (2),

each substituent group being, independently, a radical group which is a hydrocarbyl, substituted hydrocarbyl, halocarbyl, substituted halocarbyl, silylcarbyl or germylcarbyl, or Cp and Cp* are the same or different cyclopentadienyl rings in which any two adjacent substituents groups are joined to form a substituted or unsubstituted, saturated, partially unsaturated, or aromatic cyclic or polycyclic substituent; A' is a bridging group; $X_1$ and $X_2$ are, independently, hydride radicals, halide radicals, hydrocarbyl radicals, substituted hydrocarbyl radicals, halocarbyl radicals, substituted halocarbyl radicals, silylcarbyl radicals, substituted silylcarbyl radicals, germylcarbyl radicals, or substituted germylcarbyl radicals; or both X are joined and bound to the metal atom to form a metallacycle ring containing from 3 to 20 carbon atoms.

Also disclosed is a poly alpha-olefin comprised of an oligomerized alpha-olefin, wherein said oligomerized alpha-olefin is prepared by a) contacting an olefin feedstock and at least one oligomerization or polymerization catalyst to obtain an intermediate product comprising olefin trimers, wherein the olefin feedstock comprises more than 10 wt% nonene, and at least one alpha-olefin selected from the group consisting of decene, dodecane, and tetradecene, and b) separating the intermediate product to obtain a poly alpha-olefin. The oligomerization or polymerization product may be hydrogenated.

Any of the poly alpha-olefins produced from the feedstock comprising nonene may be blended with at least one other API Group I to Group V basestock.

## DETAILED DESCRIPTION OF THE INVENTION

Various specific embodiments, versions, and examples of the invention will now be described, including preferred embodiments and definitions that are adopted herein for purposes of understanding the claimed invention.

Poly alpha-olefins (PAOs) are prepared by the process of oligomerizing or polymerizing a feedstock of linear alpha-olefins in the presence of a catalyst and, optionally, at least one cocatalyst. The oligomerized or polymerized product, or a portion of the oligomerized or polymerized product, may then be hydrogenated to change the saturation level of the PAO. Further processing of the oligomerized or polymerized product or the hydrogenated product may be done to achieve the desired PAO.

### Alpha-Olefin Feedstock

The feedstock of linear alpha-olefins comprises more than 10 wt% nonene and at least one alpha-olefin selected from the group consisting of decene, dodecane, and tetradecene.

In one embodiment, the nonene is blended with decene or dodecene. The two component feedstock may be 20 wt% nonene and 80 wt% decene. Alternatively, the two component feedstock may be 40 wt% nonene and 60 wt% dodecene.

In another embodiment, the feedstock is a blend of three alpha-olefins consisting of nonene and two other alpha-olefins. Exemplary three olefin feedstocks include nonene with a combination of a) octene and decene, b) octene and dodecene, or c) decene and dodecene. When blended with two other alpha-olefins, the amount of nonene is in the range of more than 10 to 80 wt%, with the remainder of the feedstock being the other two alpha-olefins. By way of non-limited examples that are within the scope of this embodiment, the feedstock may be a) 20 wt% nonene, 60 wt% decene, and 20 wt% dodecene; b) 40 wt% nonene, 40 wt% decene, and 20 wt% dodecene; or c) 30 wt% nonene, 20 wt% octene, and 50 wt% decene.

In a preferred embodiment ethylene is present in the feed at 10 wt% or less, preferably 5 wt% or less, most preferably less than 5 wt %.

### Catalysts

The PAO fluids may be made by the oligomerization or polymerization of the feedstock alpha-olefins in the presence of an oligomerization or polymerization catalyst such as the Friedel-Crafts catalysts, including, for example $AlCl_3$, $AlBr_3$, $BF_3$, or complexes of the oligomerization or polymerization catalysts generated by a combination of the oligomerization or polymerization catalyst with at least one cocatalyst. When using only a single cocatalyst, the cocatalyst is water, an alcohol, a carboxylic acid, or an alkyl acetate. Suitable alcohols include $C_1C_{10}$ alcohols, preferably $C_1$-$C_6$ alcohols, and include methanol, ethanol, n-propanol, n-butanol, n-pentanol, and n-hexanol. Suitable acetates include $C_1$-$C_{10}$ alkyl acetates, preferably $C_1$-$C_6$ alkyl acetates including methyl acetate, ethyl acetate, n-propyl acetate, n-butyl acetate, and the like. Combinations of cocatalysts have also been determined to produce oligomers having desired physical properties and product distributions. The combination of cocatalysts includes one alcohol and at least one alkyl acetate. The cocatalyst(s) complexes with the principal catalyst to form a coordination compound which is catalytically active. The cocatalyst is used in an amount of from about 0.01 to about 10 weight percent, based on the weight of the alpha-olefin feed, most preferably about 0.1 to 6 weight percent.

Alternatively, the catalyst used in the production of the PAO, especially if the goal is the production of a high

viscosity index (HVI) PAO, may be a supported, reduced metal oxide catalyst, such as Cr compounds on silica or other supported IUPAC Periodic Table Group VIB compounds. The catalyst most preferred is a lower valence Group VIB metal oxide on an inert support. Preferred supports include silica, alumina, titania, silica alumina, magnesia and the like. The support material binds the metal oxide catalyst. Those porous substrates having a pore opening of at least 40 angstroms are preferred.

The support material usually has high surface area and large pore volumes with average pore size of 40 to about 350 angstroms. The high surface area is beneficial for supporting large amount of highly dispersive, active chromium metal centers and to give maximum efficiency of metal usage, resulting in very high activity catalyst. The support should have large average pore openings of at least 40 angstroms, with an average pore opening of 60 to 300 angstroms preferred. This large pore opening will not impose any diffusional restriction of the reactant and product to and away from the active catalytic metal centers, thus further optimizing the catalyst productivity. Also, for this catalyst to be used in fixed bed or slurry reactor and to be recycled and regenerated many times, a silica support with good physical strength is preferred to prevent catalyst particle attrition or disintegration during handling or reaction.

The supported metal oxide catalysts are preferably prepared by impregnating metal salts in water or organic solvents onto the support. Any suitable organic solvent known to the art may be used, for example, ethanol, methanol, or acetic acid. The solid catalyst precursor is then dried and calcined at 200° to 900° C by air or other oxygen-containing gas. Thereafter the catalyst is reduced by any of several various and well known reducing agents such as, for example, CO, $H_2$, $NH_3$, $H_2S$, $CS_2$, metal alkyl containing compounds such as $R_3Al$, $R_3B$, $R_2Mg$, RLi, $R_2Zn$, where R is alkyl, alkoxy, aryl and the like. Preferred are CO or $H_2$ or metal alkyl containing compounds.

Alternatively, the Group VIB metal may be applied to the substrate in reduced form, such as CrII compounds. The resultant catalyst is very active for oligomerizing or polymerizing olefins at a temperature range from below room temperature to about 250° C at a pressure of 0.1 atmosphere to 5000 psi. Contact time of both the olefin and the catalyst can vary from one second to 24 hours. The catalyst can be used in a batch type reactor or in a fixed bed, continuous-flow reactor.

In general the support material may be added to a solution of the metal compounds, e.g., acetates or nitrates, etc., and the mixture is then mixed and dried at room temperature. The dry solid gel is purged at successively higher temperatures to about 600° C for a period of about 16 to 20 hours. Thereafter the catalyst is cooled down under an inert atmosphere to a temperature of about 250° to 450° C and a stream of pure reducing agent is contacted therewith for a period when enough CO has passed through to reduce the catalyst as indicated by a distinct color change from bright orange to pale blue. Typically, the catalyst is treated with an amount of CO equivalent to a two-fold stoichiometric excess to reduce the catalyst to a lower valence CrII state. Finally the catalyst is cooled down to room temperature and is ready for use.

Alternatively, the oligomerization or polymerization reaction of the nonene containing feedstock may also be carried out in the presence of a catalyst comprising an acidic ionic liquid. Most of the ionic liquids are salts (100% ions) with a melting point below 100° C; they typically exhibit no measurable vapor pressure below thermal decomposition. The properties of ionic liquids result from the composite properties of the wide variety of cations and anions which may be present in these liquids. Many of the ionic liquids are liquid over a wide temperature range (often more than 300° C). They have low melting points (as low as -96° C has been reported), which can be attributed to large asymmetric cations having low lattice energies. As a class of materials, ionic liquids are highly solvating for both organic and inorganic materials. Depending on the ions present, ionic liquids may be neutral, basic or acidic in character. The acidic liquids will function themselves as catalysts for oligomerization or polymerization and thus may be used directly. The neutral ionic liquids will function catalytically in the present process if an additional Lewis acid component is present to confer the necessary acidity.

The acidic ionic liquid oligomerization or polymerization catalyst system will often be comprised of at least two components of which one is the ionic liquid and the other provides the desired acidic property; if, however, the ionic liquid is itself acidic, it may be used on its own as the oligomerization or polymerization catalyst. In many instances, however, the catalyst system will be a two component system with the first component being an acidic component, i.e., a Lewis acid such as an aluminum halide or an alkyl aluminum halide. For example, a typical first Lewis acid component of the catalyst system may be aluminum trichloride. The second, ionic liquid, component is advantageously a quaternary ammonium, quaternary phosphonium, or tertiary sulfonium compound, such as, for example, a liquid salt selected from one or more of hydrocarbyl substituted ammonium halides, hydrocarbyl substituted imidazolium halide, hydrocarbyl substituted pyridinium halide, hydrocarbyl substituted phosphonium halide. For example, 1-ethyl-3-methyl-imidazolium chloride can be used as a second component.

The ionic liquid is primarily a salt or mixture of salts which melts below room temperature, as noted above. Ionic liquids may be characterized by the general formula $Q^+A^-$, where is $Q^+$ is quaternary ammonium, quaternary phosphonium or tertiary sulfonium, and $A^-$ is a negatively charged ion such as $Cl^-$, $Br^-$, $OCl_4^-$, $NO_3^-$, $BF_4^-$, $BCl_4^-$, $PF_6^-$, $SbF_6^-$, $AlCl_4^-$, $CuCl_2^-$, $FeCl_3^-$.

If a two component catalyst system is used, the mole ratio of the two components of the catalyst system will

usually fall within the range of from 1:1 to 5:1 of the first (Lewis acid) component to the second (ionic liquid) component; more advantageously the mole ratio will be in the range of from 1:1 to 2:1.

In one embodiment of the ionic liquid catalyst system, the ionic liquid oligomerization or polymerization catalyst system comprises a Lewis acid component and an ionic liquid component. In another embodiment, the ionic liquid oligomerization or polymerization catalyst comprises a liquid salt selected from one or more of hydrocarbyl substituted ammonium halides, hydrocarbyl substituted imidazolium halides, hydrocarbyl substituted pyridinium halides and hydrocarbyl substituted phosphonium halides.

The catalyst system, being a liquid may also function as the solvent or diluent for the reaction so that no additional solvent or diluent is required; additional liquids which are non-reactive to the selected catalyst system may; however, be present if desired, for example, to control viscosity of the reaction mixture or to carry off heat of reaction by evaporation with reflux of the condensed vapor. Thus, the feedstock may be oligomerized or polymerized directly in the presence of the catalyst system without the addition of solvent or diluent. Since many ionic liquids are hydrocarbon soluble as a result of the presence of long chain hydrocarbon substituents, the reaction will normally proceed with a single phase reaction mixture.

The desired PAO fluids may also be oligomerized or polymerized using metallocene catalysts together with one or more activators (such as an alumoxane or a non-coordinating anion). The metallocene catalyst can be a bridged or unbridged, substituted or unsubstituted aromatic transition metal compound.

In one embodiment of a metallocene catalyst, the catalyst may be a bridged highly substituted metallocene that gives high catalyst productivity. In another embodiment, the catalyst may include bridged and substituted cyclopentadienes. In another embodiment, the catalyst may include bridged and substituted indenes or fluorenes. The metallocene compounds (pre-catalysts), useful herein are preferably cyclopentadienyl derivatives of titanium, zirconium and hafnium. In general, useful titanocenes, zirconocenes and hafnocenes may be represented by the following formula:

$$(Cp\text{-}A'\text{-}Cp^*)MX_1X_2 \qquad (1)$$

wherein: M is the metal center, and is a Group 4 metal preferably titanium, zirconium or hafnium, preferably zirconium or hafnium; Cp and Cp* are the same or different cyclopentadienyl rings that are each bonded to M, and substituted with from zero to four substituent groups S", each substituent group S" being, independently, a radical group which is a hydrocarbyl, substituted hydrocarbyl, halocarbyl, substituted halocarbyl, silylcarbyl or germylcarbyl, or Cp and Cp* are the same or different cyclopentadienyl rings in which any two adjacent S" groups are optionally joined to form a substituted or unsubstituted, saturated, partially unsaturated, or aromatic cyclic or polycyclic substituent; A' is a bridging group; $X_1$ and $X_2$ are, independently, hydride radicals, hydrocarbyl radicals, substituted hydrocarbyl radicals, halocarbyl radicals, substituted halocarbyl radicals, silylcarbyl radicals, substituted silylcarbyl radicals, germylcarbyl radicals, or substituted germylcarbyl radicals; or both X are joined and bound to the metal atom to form a metallacycle ring containing from about 3 to about 20 carbon atoms; or both together can be an olefin, diolefin or aryne ligand; or when Lewis-acid activators, such as methylalumoxane, which are capable of donating an X ligand as described above to the transition metal component are used, both X may, independently, be a halogen, alkoxide, aryloxide, amide, phosphide or other univalent anionic ligand or both X can also be joined to form an anionic chelating ligand. In a preferred embodiment, the metallocene is racemic wherein the compound has no plane of symmetry containing the metal center, M; and has a $C_2$-axis of symmetry or pseudo $C_2$-axis of symmetry through the metal center.

Alternatively, feedstocks may be oligomerized by means of unbridged, substituted aromatic transition metal compounds with one or more non-coordinating anion activators or alumoxane activators. Embodiments of such catalysts include unbridged and substituted cyclopentadienes, unbridged and substituted or unsubstituted indenes, and unbridged and substituted or unsubstituted fluorenes.

In another embodiment of the unbridged transition metal compound, the unbridged, substituted aromatic transition metal compound has: 1) at least one non-isoolefin substitution on each ring, or 2) at least two substitutions on at least one ring, preferably having at least two substitutions on each ring. The unbridged transition metal compound has the following formula:

$$(CpCp^*)MX_1X_2 \qquad (2)$$

wherein Cp, Cp*, M, $X_1$, and $X_2$ have the same structures as described above in reference to formula (1), except that Cp and Cp* may be substituted with zero to five substituents S".

In a preferred embodiment, when using a metallocene catalyst to obtain a low viscosity PAO, the transition metal has the following structure:

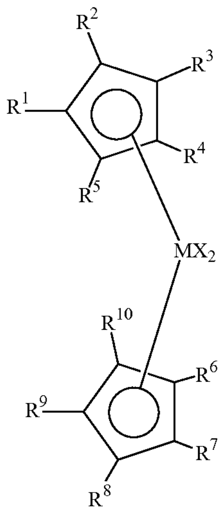

where M is a Group 4 metal preferably titanium, zirconium or hafnium, preferably zirconium or hafnium, each X is a hydrogen, halogen, hydride radicals, hydrocarbyl radicals, substituted hydrocarbyl radicals, halocarbyl radicals, substituted halocarbyl radicals, silylcarbyl radicals, substituted silylcarbyl radicals, germylcarbyl radicals, or substituted germylcarbyl radicals; or both X are joined and bound to the metal atom to form a metallacycle ring containing from about 3 to about 20 carbon atoms; or both together can be an olefin, diolefin or aryne ligand; and $R^1$ to $R^{10}$ are independently, a radical group which is a hydrocarbyl, substituted hydrocarbyl, halocarbyl, substituted halocarbyl, silylcarbyl or germylcarbyl, (preferably hydrogen, or a $C_1$ to $C_{20}$ hydrocarbyl, a substituted $C_1$ to $C_{20}$ hydrocarbyl, or a heteroatom), provided that: 1) at least one of $R^1$ to $R^5$ is not hydrogen or an isoolefin and at least one of $R^6$ to $R^{10}$ is not hydrogen or an isoolefin, or 2) at least two of $R^1$ to $R^5$ are not hydrogen, (and preferably at least two of $R^6$ to $R^{10}$ are not hydrogen) where any two adjacent $R^1$ to $R^5$ groups may form a $C_4$ to $C_{20}$ cyclic or polycyclic moiety (such as substituted or unsubstituted indene or substituted or unsubstituted fluorene), and where any two adjacent $R^6$ to $R^{10}$ groups may form a $C_4$ to $C_{20}$ cyclic or polycyclic moiety (such as substituted or unsubstituted indene or substituted or unsubstituted fluorene).

The catalyst precursors, when activated by a commonly known activator such as methylalumoxane, form active catalysts for the polymerization or oligomerization of olefins. Activators that may be used include alumoxanes such as methylalumoxane, modified methylalumoxane, ethylalumoxane, *iso*-butylalumoxane and the like; Lewis acid activators include triphenylboron, tris-perfluorophenylboron, tris-perfluorophenylaluminum and the like; ionic activators include dimethylanilinium tetrakisperfluorophenylborate, triphenylcarboniumtetrakis perfluorophenylborate, dimethylanilinium tetrakisperfluorophenylaluminate, and the like.

A co-activator is a compound capable of alkylating the transition metal complex, such that when used in combination with an activator, an active catalyst is formed. Co-activators include alumoxanes such as methylalumoxane, modified alumoxanes such as modified methylalumoxane, and aluminum alkyls such trimethylaluminum, tri-isobutylaluminum, triethylaluminum, and tri-isopropylaluminum, tri-n-hexylaluminum, tri-n-octylaluminum, tri-n-decylaluminum or tri-n-dodecylaluminum. Co-activators are typically used in combination with Lewis acid activators and ionic activators when the pre-catalyst is not a dihydrocarbyl or dihydride complex. Sometimes co-activators are also used as scavengers to deactivate impurities in feed or reactors.

Particularly preferred co-activators include alkylaluminum compounds and are represented by the formula: $R_3Al$, where each R is, independently, a $C_1$ to $C_{18}$ alkyl group, preferably each R is, independently, selected from the group consisting of methyl, ethyl, n-propyl, iso-propyl, iso-butyl, n-butyl, t-butyl, n-pentyl, iso-pentyl, neopentyl, n-hexyl, isohexyl, n-heptyl, iso-heptyl, n-octyl, iso-octyl, n-nonyl, n-decyl, n-undecyl, n-dodecyl, n-tridecyl, n-tetradecyl, n-pentadecyl, n-hexadecyl, n-heptadecyl, n-octadecyl, and their iso-analogs.

The alumoxane component useful as an activator typically is preferably an oligomeric aluminum compound represented by the general formula $(R^x\text{-Al-O})_n$, which is a cyclic compound, or $R^x$ $(R^x\text{-Al-O})_nAlR^x{}_2$, which is a linear compound. It is believed that the most common alumoxanes are a mixture of the cyclic and linear compounds. In the general alumoxane formula, $R^x$ is independently a $C_1$-$C_{20}$ alkyl radical, for example, methyl, ethyl, propyl, butyl, pentyl, isomers thereof, and the like, and "n" is an integer from 1-50. Most preferably, $R^x$ is methyl and "n" is at least 4. Methylalumoxane and modified methylalumoxanes are most preferred.

When an alumoxane or modified alumoxane is used, the catalyst-precursor-to-activator molar ratio is from about 1:3000 to 10:1; alternatively, 1:2000 to 10:1; alternatively 1:1000 to 10:1; alternatively, 1:500 to 1:1; alternatively 1:300 to 1:1; alternatively 1:250 to 1:1, alternatively 1:200 to 1:1; alternatively 1:100 to 1:1; alternatively 1:50 to 1:1; alternatively 1:10 to 1:1. When the activator is an alumoxane (modified or unmodified), some embodiments select the

maximum amount of activator at a 5000-fold molar excess over the catalyst precursor (per metal catalytic site). The preferred minimum activator-to-catalyst-precursor ratio is 1:1 molar ratio.

Ionic activators (at times used in combination with a co-activator) may be used in the practice of this invention. Preferably, discrete ionic activators such as $[Me_2PhNH][B(C_6F_5)_4]$, $[Ph_3C][B(C_6F_5)_4]$, $[Me_2PhNH][B((C_6H_3\text{-}3,5\text{-}(CF_3)_2))_4]$, $[Ph_3C][B((C_6H_3\text{-}3,5\text{-}(CF_3)_2))_4]$, $[NH_4][B(C_6H_5)_4]$ or Lewis acidic activators such as $B(C_6F_5)_3$ or $B(C_6H_5)_3$ can be used, where Ph is phenyl and Me is methyl. Preferred co-activators, when used, are alumoxanes such as methylalumoxane, modified alumoxanes such as modified methylalumoxane, and aluminum alkyls such as tri-isobutylaluminum, and trimethylaluminum, triethylaluminum, and tri-isopropylaluminum, tri-n-hexylaluminum, tri-n-octylaluminum, tri-n-decylaluminum or tri-n-dodecylaluminum.

Supported catalysts and or supported catalyst systems may be used to prepare PAO's. Useful supported catalyst systems may be prepared by any method effective to support other coordination catalyst systems, effective meaning that the catalyst so prepared can be used for oligomerizing or polymerizing olefins in a heterogeneous process. The catalyst precursor, activator, co-activator (if needed), suitable solvent, and support may be added in any order or simultaneously. Additionally, two or more different catalyst precursors may be placed on the same support. Likewise, two or more activators or an activator and a co-activator, may be placed on the same support.

Suitable solid particle supports are typically comprised of polymeric or refractory oxide materials, each being preferably porous. Any support material that has an average particle size greater than 10 μm is suitable for use in this invention. Various embodiments select a porous support material, such as, for example, talc, inorganic oxides, inorganic chlorides, for example magnesium chloride and resinous support materials, such as polystyrene polyolefin or polymeric compounds or any other organic support material and the like. Some embodiments select inorganic oxide materials as the support material including Group-2, -3, -4, -5, -13, or -14 metal or metalloid oxides. Some embodiments select the catalyst support materials to include silica, alumina, silica-alumina, and their mixtures. Other inorganic oxides may serve either alone or in combination with the silica, alumina, or silica-alumina. These are magnesia, titania, zirconia, and the like. Lewis acidic materials such as montmorillonite and similar clays may also serve as a support. In this case, the support can optionally double as an activator component. But additional activator may also be used. In some cases, a solid crystalline support can also be used. The crystalline support can be prepared with tunable pore size and tunable acidity when modified with a second component; MCM-41 is one example of such a crystalline support. A detailed description of this class of materials and their modification can be found in US 5,264,203. Polymeric carriers for the metallocene catalyst will also be suitable for use in oligomerizing or polymerizing the nonene containing feedstock.

Useful catalyst carriers may have a surface area of from 10-700 $m^2/g$, and or a pore volume of 0.1-4.0 cc/g and or an average particle size of 10-500 μm. Some embodiments select a surface area of 50-500 $m^2/g$, and or a pore volume of 0.5-3.5 cc/g, and or an average particle size of 20-200 μm. Other embodiments select a surface area of 100-400 $m^2/g$, and or a pore volume of 0.8-3.0 cc/g, and or an average particle size of 30-100 μm Invention carriers typically have a pore size of 10-1000 Angstroms, alternatively 50-500 Angstroms, or 75-350 Angstroms. The metallocenes and or the metallocene/activator combinations are generally deposited on the support at a loading level of 10-100 micromoles of catalyst precursor per gram of solid support; alternately 20-80 micromoles of catalyst precursor per gram of solid support; or 40-60 micromoles of catalyst precursor per gram of support. But greater or lesser values may be used provided that the total amount of solid catalyst precursor does not exceed the support's pore volume.

In another embodiment, prior to entering the reactor, the metallocene, the activator (with or without a support), or the feedstream are combined with a poison scavenger to improve catalyst efficiency. The scavenger is an alkylaluminum compound represented by the formula: $R_3Al$, where each R is independently a $C_1$ to $C_{20}$ alkyl group; preferably the R groups are independently selected from the group consisting of methyl, ethyl, propyl, isopropyl, butyl, isobutyl, n-butyl, pentyl, isopentyl, n-pentyl, hexyl, isohexyl, n-hexyl, heptyl, octyl, isooctyl, n-octyl, nonyl, isononyl, n-nonyl, decyl, isodecyl, n-decyl, undecyl, isoundecyl, n-undecyl, dodecyl, isododecyl, and n-dodecyl, preferably isobutyl, n-octyl, n-hexyl, and n-dodecyl. Preferably the alkylaluminum compound is selected from tri-isobutyl aluminum, tri n-octyl aluminum, tri-n-hexyl aluminum, and tri-n-dodecyl aluminum. The molar ratio of transition metal compound to activator is 10:1 to 0.1:1, and if the scavenger compound is present then the molar ratio of scavenger compound to transition metal compound is 1:4 to 4000:1.

Further examples of suitable metallocene catalysts and activators for the catalysts may be found in US 2007/0043248 and US 2009/005279.

**Process of Manufacture**

In the following description of manufacturing the poly alpha-olefin, the terms oligomerized and polymerized, and derivatives of both words, are used; for the purpose of this disclosed invention, oligomerized and polymerized are synonymous and indicate a reaction wherein the individual polymers are bonded together via a chemical reaction. The nonene or mixture of nonene and alphaolefins is polymerized continuously using the selected catalyst in at least one continuously stirred reactor. Monomers, dimers, and catalyst are removed from the reaction mixture and may be recovered

and reused. In embodiments, for instance in the case where the dimers are a desired product, the product is preferably first hydrogenated prior to distillation of the dimers. If the dimers are removed first, the product is then hydrogenated to saturate oligomers. The final product may then be distilled further to produce, in some embodiments, different grades of PAO.

The reaction may be batch, semi-batch or continuous, in a single or multi-stage reactors. The reaction zone may be any reaction means known in the art that provides for the reaction under suitable conditions maintained and controlled so as to provide for the production of oligomers of the feedstock. It is preferred that the reactors each be equipped with a mixing or stirring means for mixing the feed and catalyst to provide intimate contact. In a more preferred embodiment, continuous stirred tank reactors (CSTRs) are used in series. CSTRs are per se known in the art.

The feedstock, catalyst, and any cocatalysts may be introduced either separately or together into the first reaction zone. In one embodiment, the catalyst is introduced into the reactor simultaneously with any cocatalysts and the olefin feedstock. The catalyst can be sparged into the reaction mixture, along with other known methods for introducing the catalyst to the reaction zone. In the case of more than one continuously stirred reactor connected in series, in another embodiment, the catalyst, cocatalyst and olefin feed are introduced only to the first reactor, and preferably simultaneously. In another embodiment, the mixture of catalyst and olefin feedstock is fed into a first oligomerization reactor where it is partially reacted and then into a second oligomerization reactor where the reaction may be allowed to continue to completion or where the reaction may be allowed to proceed further and then the mixture of catalyst, linear alpha-olefins and oligomers are fed into a third oligomerization reactor where the reaction is completed. Additional oligomerization reactors may be used in series.

Reaction conditions are such as to cause effective conversion of monomers to the desired product. Such conditions may also be determined by one of ordinary skill in the art in possession of the present disclosure without undue experimentation. It is generally most economical to operate the reactors at a low pressure, preferably from about atmospheric to about 50 psia. In one embodiment, the reaction zone(s) contain an excess of catalyst, which is governed by the pressure and partial pressure of the catalyst. In this regard, it is preferred that the catalyst be maintained in the reaction zone at a pressure of about 2 to about 500 psig, preferably about 2 to 50 psig (1 psi=703 kg/$m^2$).

Suitable temperatures for the reaction are also conventional and can vary from about -20° C to about 90° C, with a range of about 15° to 70° C being preferred. Appropriate residence times in each reactor, and other further details of processing, are within the skill of the ordinary artisan, in possession of the present disclosure.

In one embodiment, no solvent is used. In another embodiment, an inert diluent may be used, preferably selected from fluids such as $C_5$-$C_{19}$ paraffinic hydrocarbons, preferably a $C_6$-$C_{13}$ paraffinic fluid such as Norpar™ 12 fluid, an aliphatic (paraffinic) solvent having primarily twelve carbon aliphatic compounds.

If metallocene or supported, reduced metal oxide catalysts are employed, prior to introduction of the feedstock into the reactor, the feedstock may be treated to remove catalyst poisons, such as peroxides, oxygen, sulfur, nitrogen-containing organic compounds, and or acetylenic compounds. This treatment is believed to increase catalyst productivity, typically more than 5 fold, preferably more than 10 fold.

When employing a metallocene catalyst for producing the PAO's many polymerization/oligomerization processes and reactor types used for metallocene-catalyzed polymerizations or oligomerizations such as solution, slurry, and bulk polymerization or oligomerization processes can be used in this invention. In some embodiments, if a solid or supported catalyst is used, a slurry or continuous fixed bed or plug flow process is suitable. In a preferred embodiment, the monomers are contacted with the metallocene compound and the activator in the solution phase, bulk phase, or slurry phase, preferably in a continuous stirred tank reactor, continuous tubular reactor, or a batch reactor.

The temperature in any reactor used for metallocene catalyst production is from - 10 °C to 250 °C, preferably from 30 °C to 220 °C, preferably from 50 °C to 180 °C, preferably from 60 °C to 170 °C. The pressure in any reactor used herein is from 0.1 to 100 atmospheres, preferably from 0.5 to 75 atmospheres, preferably from 1 to 50 atmospheres. In another embodiment, the pressure in any reactor used herein is from 1 to 50,000 atmospheres, preferably 1 to 25,000 atmospheres. In another embodiment, the monomer(s), metallocene and activator are contacted for a residence time of 1 second to 100 hours, preferably 30 seconds to 50 hours, preferably 2 minutes to 6 hours, preferably 1 minute to 4 hours. In another embodiment solvent or diluent is present in the reactor and is preferably selected from the group consisting of butanes, pentanes, hexanes, heptanes, octanes, nonanes, decanes, undecanes, dodecanes, tridecanes, tetradecanes, pentadecanes, hexadecanes, toluene, o-xylene, m-xylene, p-xylene, ethylbenzene, isopropylbenzene, and n-butylbenzene; preferably toluene and or xylenes and or ethylbenzene, normal paraffins, or isoparaffin solvents ( such as Isopar solvents available from ExxonMobil Chemical Company in Houston, Texas). These solvents or diluents are usually pre-treated in same manners as the feed olefins.

In the processes of this embodiment, one or more transition metal compounds, optionally, one or more activators, and one or more monomers are contacted to produce polymer or oligomer. These catalysts may be supported and as such will be particularly useful in the known slurry, solution, or bulk operating modes conducted in single, series, or parallel reactors. If the catalyst, activator or co-activator is a soluble compound, the reaction can be carried out in a solution mode. Even if one of the components is not completely soluble in the reaction medium or in the feed solution,

either at the beginning of the reaction or during or at the later stages of the reaction, a solution or slurry type operation is still applicable. In any instance, the catalyst components, dissolved or suspended in solvents, such as toluene or other conveniently available aromatic solvents, or in aliphatic solvent, or in the feed alpha-olefin stream, are fed into the reactor under inert atmosphere (usually nitrogen or argon blanketed atmosphere) to allow the polymerization or oligomerization to take place. The polymerization or oligomerization can be run in a batch mode, where all the components are added into a reactor and allowed to react to a pre-designed degree of conversion, either to partial conversion or full conversion. Subsequently, the catalyst is deactivated by any possible means, such as exposure to air or water, or by addition of alcohols or solvents containing deactivating agents. The polymerization or oligomerization can also be carried out in a semi-continuous operation, where feeds and catalyst system components are continuously and simultaneously added to the reactor so as to maintain a constant ratio of catalyst system components to feed olefin(s). When all feeds and catalyst components are added, the reaction is allowed to proceed to a pre-determined stage. The reaction is then discontinued by catalyst deactivation in the same manner as described for batch operation. The polymerization or oligomerization can also be carried out in a continuous operation, where feeds and catalyst system components are continuously and simultaneously added to the reactor so to maintain a constant ratio of catalyst system and feed olefins. The reaction product is continuously withdrawn from the reactor, as in a typical continuous stirred tank reactor (CSTR) operation. The residence times of the reactants control the degree of conversion. The withdrawn product is then typically quenched in the separate reactor in a similar manner as other operation. In a preferred embodiment, any of the processes to prepare PAO's described herein are continuous processes. Preferably the continuous process comprises the steps of a) continuously introducing a feed stream comprising at least 10 mole % of the feedstock alpha-olefins into a reactor, b) continuously introducing the metallocene compound and the activator into the reactor, and c) continuously withdrawing the poly alpha-olefin from the reactor. In another embodiment, the continuous process comprises the step of maintaining a partial pressure of hydrogen in the reactor of 200 psi (1379 kPa) or less, based upon the total pressure of the reactor, preferably 150 psi (1034 kPa) or less, preferably 100 psi (690 kPa) or less, preferably 50 psi (345 kPa) or less, preferably 25 psi (173 kPa) or less, preferably 10 psi (69 kPa) or less. Alternately, the hydrogen, if present, is present in the reactor at 1000 ppm or less by weight, preferably 750 ppm or less, preferably 500 ppm or less, preferably 250 ppm or less, preferably 100 ppm or less, preferably 50 ppm or less, preferably 25 ppm or less, preferably 10 ppm or less, preferably 5 ppm or less. Alternately, the hydrogen, if present, is present in the feed at 1000ppm or less by weight, preferably 750 ppm or less, preferably 500 ppm or less, preferably 250 ppm or less, preferably 100 ppm or less, preferably 50 ppm or less, preferably 25 ppm or less, preferably 10 ppm or less, preferably 5 ppm or less.

Just as with non-metallocene catalysts, when manufacturing the PAO using a metallocene catalyst, one or more reactors in series or in parallel may be used or a single reactor may be used.

The reaction time or reactor residence time is usually dependent on the type of catalyst used, the amount of catalyst used, and the desired conversion level.

## PAO

The PAO's produced according to this invention are typically dimers, trimers, tetramers, or higher oligomers or polymers of the nonene in combination with the one or more olefin monomers selected from the group consisting of decene, dodecane, and tetradecene. Alternatively, an alpha-olefin with alkyl substitutent at least 2 carbons away from the olefinic double bond can also be used. Typically, the PAO's produced herein are usually a mixture of many different oligomers. The smallest oligomers from these alpha-olefins are generally dimers of the feedstock olefins. These small oligomers are usually too light for most high performance fluids applications. They are usually separated from the higher oligomers with carbon number of greater than $C_{18}$ or $C_{20}$, for example $C_{24}$ and higher which are more preferred as high performance fluids. These separated oligomer olefins or the corresponding paraffins after hydrogenation can be used in specialty applications, such as drilling fluids, solvents, paint thinner, etc. with excellent biodegradability, toxicity, viscosities, etc. The high performance fluid fraction in the $C_{20}$, or $C_{30}$ and higher fractions typically have lower viscosities making them beneficial for some applications, such as better fuel economy, better biodegradability, better low temperature flow properties, or lower volatility. The higher viscosity products usually have a much higher average degree of polymerization and have very low amounts of C20 or C30 component. These high viscosity fluids are excellent blend stocks for lube applications to improve the viscosity. Because of their usually narrow molecular weight distribution, they have superior shear stability. Because of their unique chemical composition, they have excellent viscometrics and unexpected low traction properties. These higher viscosity PAOs can be used as superior blend stocks. They can be blend stocks with any of the Gr I, II, III, III+, GTL and Gr V fluids to give the optimum viscometrics, solvency, high and low temperature lubricity, etc. They can also be further blended with proper additives, including antioxidants, antiwear additives, friction modifiers, dispersants, detergents, corrosion inhibitors, defoamants, extreme pressure additives, seal swell additives, and optionally viscosity modifiers, etc. In one embodiment, the PAO has not more than 20 wt% of dimers. In another embodiment, the PAO has at lest 50 wt% of trimers and tetramers of the feedstock olefins.

The PAOs produced by the disclosed feedstock comprising nonene and at least one alpha-olefin selected from

the group consisting of decene, dodecane, and tetradecene, in any combination with any of the above catalysts and processes, will have a kinematic viscosity, measured at 100° C, ranging from 1.5 cSt to 2,000 cSt. When the PAO has a 100° C viscosity of less than 40 cSt, the PAO is generally referred to as a low viscosity PAO. When the PAO has a 100° C viscosity of 40 cSt or greater, the PAO is generally referred to as a high viscosity PAO.

Other possible PAOs that may be produced using the disclosed feeds include what are referred to as high Viscosity Index (HVI) PAOs. Such HVI PAOs have viscosities at 100° C of at least 100 cSt. The Viscosity Index for HVI PAOs will be greater than 200.

**Exemplary PAOs**

The following examples are meant to illustrate embodiments of the present invention, and it will be recognized by one of ordinary skill in the art in possession of the present disclosure that numerous modifications and variations are possible. Therefore, it is to be understood that within the scope of the appended claims, the invention may be practiced otherwise than as specifically described herein.

For each of the reported examples below, 100 °C, 40 °C, and -40 °C Kinematic Viscosity was measured with reference to ASTM D-445 at the respective temperatures; Pour Point was determined with reference to ASTM D-97; Viscosity Index (VI) was determined with reference to ASTM D-2270; and Noack Volatility was determined with reference to ASTM D-5800 Procedure B. The following abbreviations were used: BuOH = 1-Butanol; BuAc = Butyl acetate; PrOH = 1-Propanol; PeOH = 1-Pentanol.

Comparative Example A and Examples B-E

The mixture of LAOs is oligomerized either by semi-batch mode in a single stirred tank reactor or by continuous mode in a series of stirred tank reactors using $BF_3$ and $BF_3$ promoted with a mixture of normal alcohol and acetate. In semi-batch mode, the reaction mixture is quenched with 0.5 wt % aqueous NaOH and washed with distilled water. The separated product is distilled to remove the unreacted monomers and dimers. In continuous mode, the reaction mixture is combined with excess alcohol and distilled to remove promoter and unreacted monomers and dimers. The resulting products are hydrogenated to saturate the oligomers. Reaction conditions and product properties are given in Table 1.

Table 1

| Example | Comparative Example A* | Example B* | Example C | Example D** | Example E |
|---|---|---|---|---|---|
| Feedstock, wt:wt | C9 100 | C9:C10 47:53 | C9:C10 25:75 | C9:C10 35:65 | C9:C10 50:50 |
| Catalyst | $BF_3$-BuOH/BuAc | $BF_3$-BuOH/BuAc | $BF_3$-PrOH | $BF_3$-PrOH | $BF_3$-PeOH |
| Catalyst, (mmols/100 g LAO) | 30 | 30 | 20 | 30 | 20 |
| Temp. °C | 32 | 32 | 25 | 25 | 25 |
| Pressure (psig) | 5 | 5 | 5 | 5 | 5 |
| Conversion %, C18+ | 94 | 95 | 99 | 98 | 96 |
| Yield %, C27+ | 65 | 62 | 96 | 92 | 76 |
| 100 °C, cSt | 3.0 | 3.2 | 5.1 | 5.0/5.2 | 5.1 |
| 40 °C, cSt | 11.9 | 13.1 | 26.7 | 26.8/28.4 | 28.8 |
| -40 °C, cSt | 1341 | 1603 | 9145 | 11208/12446 | 17740 |
| VI | 109 | III | 119 | 112/114 | 101 |
| Pour Point, °C | -78 | -78 | ≤-60 | ≤-60/≤-60 | -- |

(continued)

| Example | Comparative Example A* | Example B* | Example C | Example D** | Example E |
|---|---|---|---|---|---|
| Noack Volatility, % | 31 | 23.1 | 16.4 | 17.0/14.0 | 20.2 |
| -- property not determined<br>* properties determined for C27-C30 fraction<br>** properties determined on two different samples | | | | | |

Comparative Examples F and H and Examples G and I

Oligomerization reactions were carried out in a four-neck 12 liter round bottom jacketed glass flask (reactor) that was fitted with a motor driven stirrer and a baffle. A pump circulated water from a temperature bath through the jacket to control reaction temperature. The LAO feed mixture was charged into a feed vessel. In some cases, a n-paraffin (obtained from Sasol Solvents) was added to the olefin mixture (28 wt% of olefins) to improve mixing and heat transfer during the oligomerization, and m-xylene (0.5 wt% of olefins) was added to improve the oligomerization of LAOs. Dry nitrogen was used to purge the reactor to remove moisture before the start of oligomerization. Subsequently, a small amount of nitrogen was added during the reaction. The desired amount of $AlCl_3$ catalyst (obtained from Gulbrandsen Chemicals), typically 0.8 to 3.5 wt% of feed, was pre-weighed and stored in closed glass vials under nitrogen. At the start of oligomerization, feed olefin mixture was pumped into the flask under vigorous agitation with the stirrer set at 725-750 rpm. A measured amount of DI (deionized) water used as a proton donor was pumped into the reaction flask at a setting of 0.45-0.5 moles of water per mole of $AlCl_3$. The LAO, $AlCl_3$, and water were added to the reaction flask over the course of 3 hours. After 3 hours, the reactor was allowed to hold for one more hour without LAO feed, water or catalyst. Thereafter, the reaction was quenched by adding into the reactor contents an equal volume of caustic (10 wt% aqueous sodium hydroxide) solution at 65 °C. The quenched mass was subsequently washed two times with hot water at 65 °C. The separated product was distilled to remove unreacted monomer and dimer and then hydrogenated to saturate the oligomers. The reactor conditions and product properties are summarized in Table 2.

Table 2

| Example | Comparative Example F | Example G | Comparative Example H | Example I |
|---|---|---|---|---|
| Feedstock, wt:wt | C9 100 | C9:C12 50:50 | C9 100 | C9:C12 50:50 |
| Catalyst | $AlCl_3$ | $AlCl_3$ | $AlCl_3$ | $AlCl_3$ |
| Catalyst Conc., (wt. % of total liquid feed) | 1.1 | 1.2 | 2.45 | 2.3 |
| n-Paraffin (wt. % of olefins) | 0 | 0 | 28 | 28 |
| m-xylene (wt. % of olefins) | 0 | 0 | 0.5 | 0.5 |
| Temp. °C | 50 | 50 | 40 | 40 |
| $H_2O$:$AlCl_3$ mole ratio | 0.5:1 | 0.5:1 | 0.5:1 | 0.5:1 |
| Conversion %, C18+ | 96.6 | 96.2 | 97.3 | 89 |
| Yield %, C27+ | 96 | 92 | 97 | 89 |
| 100 °C, cSt | 39.4 | 41.5 | 99 | 102 |
| 40 °C, cSt | 411 | 417 | 1310 | 1245 |
| Brookfield Viscosity @ -26 °C, cP | 119500 | nm | nm | 456000 |
| VI | 144 | 151 | 163 | 172 |
| Pour Point, °C | -43 | -43 | -31 | -34 |

## Industrial Applicability

The invention, accordingly, provides the following embodiments:

A. A process for the oligomerization or polymerization of alpha-olefins, the process comprising:

a) contacting a feedstock of alpha-olefins and at least one oligomerization or polymerization catalyst system in a reactor under oligomerization or polymerization conditions to oligomerize or polymerize the alpha-olefins, the feedstock of alpha-olefins comprising more than 10 wt% nonene and at least one alpha-olefin selected from the group consisting of decene, dodecane, and tetradecene;
b) removing unreacted alpha-olefins to obtain a bottoms product; and
c) optionally hydrogenating the bottoms product to obtain a hydrogenated product;

B. The process of embodiment A, wherein the feedstock consists of from more than 10 to 80% nonene and at least two alpha-olefins selected from the group consisting of decene, dodecene, and tetradecene;
C. The process of any one of embodiments A or B, wherein the process includes the further step of separating the hydrogenated product to obtain at least two fractions of poly alpha-olefins of differing nominal viscosities;
D. The process of any one of embodiments A to C, wherein the catalyst system comprises a catalyst and the catalyst is selected from the group consisting of a Friedel-Crafts catalyst, a supported reduced metal oxide catalyst, an acidic ionic liquid, a bridged substituted aromatic transition metal compound, and an unbridged substituted aromatic transition metal compound;
E. The process of any one of embodiments A to C, wherein the catalyst system comprises a catalyst and the catalyst is selected from the group consisting of $AlCl_3$, $AlBr_3$, $BF_3$, a compound represented by the formula (1) (Cp-A'-Cp*)$MX_1X_2$, and a compound represented by the formula (2) (CpCp*)$MX_1X_2$ wherein M is a metal center; Cp and Cp* are the same or different cyclopentadienyl rings that are each bonded to M, and substituted with from zero to four substituent groups for formula (1) and zero to five substituents for formula, (2) each substituent group being, independently, a radical group which is a hydrocarbyl, substituted hydrocarbyl, halocarbyl, substituted halocarbyl, silylcarbyl or germylcarbyl, or Cp and Cp* are the same or different cyclopentadienyl rings in which any two adjacent substituents groups are joined to form a substituted or unsubstituted, saturated, partially unsaturated, or aromatic cyclic or polycyclic substituent; A' is a bridging group; $X_1$ and $X_2$ are, independently, hydride radicals, halide radicals, hydrocarbyl radicals, substituted hydrocarbyl radicals, halocarbyl radicals, substituted halocarbyl radicals, silylcarbyl radicals, substituted silylcarbyl radicals, germylcarbyl radicals, or substituted germylcarbyl radicals; or both X are joined and bound to the metal atom to form a metallacycle ring containing from 3 to 20 carbon atoms;
F. The process of any one of embodiments A to E, wherein the bottoms product is a poly alpha-olefin having a kinematic viscosity in the range of 1.5 to 2000 cSt at 100° C;
G. The process of any one of embodiments A to F, , wherein the bottoms product is a poly alpha-olefin comprising not more than 20 wt% of oligomers having a carbon count in the range of $C_{18}$ to $C_{20}$;
H. The process of any one of embodiments A to G, wherein the bottoms product is a poly alpha-olefin comprising at least 50 wt% of oligomers having a carbon count of in the range of $C_{27}$ to $C_{40}$;
I. A poly alpha-olefin comprised of an oligomerized alpha-olefin, wherein said oligomerized alpha-olefin is prepared by a) contacting an olefin feedstock and at least one oligomerization or polymerization catalyst to obtain an intermediate product comprising olefin trimers, wherein the olefin feedstock comprises more than 10 wt% nonene and at least one alpha-olefin selected from the group consisting of decene, dodecane, and tetradecene, and b) separating the intermediate product to obtain a bottoms product;
J. The poly alpha-olefin of any one of embodiment I, wherein the poly alpha-olefin has been hydrogenated;
K. The poly alpha-olefin of any one of embodiments I or J, wherein the bottoms product is a poly alpha-olefin having a kinematic viscosity in the range of 1.5 to 2000 cSt at 100° C;
L. The poly alpha-olefin of any one of embodiments I to K, wherein the bottoms product is a poly alpha-olefin comprising not more than 20 wt% of oligomers having a carbon count in the range of C18 to C20;
M. The poly alpha-olefin of any one of embodiments I to L, wherein the bottoms product is a poly alpha-olefin comprising at least 50 wt% of oligomers having a carbon count of in the range of C27 to C40; and
N. The poly alpha-olefin of any one of embodiment I to M, wherein the poly alpha-olefin is further blended with at least one additional API Group I to Group V basestock.

The PAOs produced as taught herein are useful herein by themselves as lubricants or functional fluids, or they may be mixed with various conventional additives. They may also be blended with other basestocks, such as American Petroleum Institute (API) Groups I to III and V, or other conventional PAOs (API Group IV), and also other hydrocarbon fluids, e.g., isoparaffins, normal paraffins, and the like. When formulating lubricants with the PAOs, the PAOs, other

basestocks, and other hydrocarbon fluids may form a major or minor portion of the overall lubricant composition and the choice thereof and quantity, as well as any additional additives, can be tailored to meet desired end-use criteria.

## Claims

1. A process for the oligomerization of alpha-olefins, the process comprising:

   a) contacting a feedstock of alpha-olefins and at least one oligomerization or polymerization catalyst system in a reactor under oligomerization or polymerization conditions to oligomerize or polymerize the alpha-olefins, the feedstock of alpha-olefins comprising more than 10 wt% nonene, and at least one alpha-olefin selected from the group consisting of decene, dodecene, and tetradecene;
   b) removing unreacted alpha-olefins to obtain a bottoms product; and
   c) optionally hydrogenating the bottoms product to obtain a hydrogenated product.

2. The process of claim 1, wherein the feedstock consists of from more than 10 to 80 wt% nonene and at least two alpha-olefins selected from the group consisting of decene, dodecene, and tetradecene.

3. The process of claim 1 or 2 wherein the process includes the further step of separating the hydrogenated product to obtain at least two fractions of poly alpha-olefins of differing nominal viscosities.

4. The process of any one of claims 1 to 3, wherein the catalyst system comprises a catalyst and the catalyst is selected from the group consisting of a Friedel-Crafts catalyst, a supported reduced metal oxide catalyst, an acidic ionic liquid, a bridged substituted aromatic transition metal compound, and an unbridged substituted aromatic transition metal compound.

5. The process of any one of claims 1 to 4, wherein the catalyst system comprises a catalyst and the catalyst is selected from the group consisting of $AlCl_3$, $AlBr_3$, $BF_3$, a compound represented by the formula (1) (Cp-A'-Cp*)$MX_1X_2$, and a compound represented by the formula (2) (CpCp*)$MX_1X_2$ wherein M is a metal center; Cp and Cp* are the same or different cyclopentadienyl rings that are each bonded to M, and substituted with from zero to four substituent groups for formula (1) and zero to five substituents for formula (2), each substituent group being, independently, a radical group which is a hydrocarbyl, substituted hydrocarbyl, halocarbyl, substituted halocarbyl, silylcarbyl or germylcarbyl, or Cp and Cp* are the same or different cyclopentadienyl rings in which any two adjacent substituents groups are joined to form a substituted or unsubstituted, saturated, partially unsaturated, or aromatic cyclic or polycyclic substituent; A' is a bridging group; $X_1$ and $X_2$ are, independently, hydride radicals, halide radicals, hydrocarbyl radicals, substituted hydrocarbyl radicals, halocarbyl radicals, substituted halocarbyl radicals, silylcarbyl radicals, substituted silylcarbyl radicals, germylcarbyl radicals, or substituted germylcarbyl radicals; or both X are joined and bound to the metal atom to form a metallacycle ring containing from 3 to 20 carbon atoms.

6. The process of any one of claims 1 to 5, wherein the bottoms product is a poly alpha-olefin having a kinematic viscosity in the range of 1.5 to 2000 cSt at 100 °C.

7. The process of any one of claims 1 to 6, wherein the bottoms product is a poly alpha-olefin comprising at least 50 wt % of oligomers having a carbon count of in the range of $C_{27}$ to $C_{40}$.

8. A poly alpha-olefin comprised of an oligomerized alpha-olefin, wherein said oligomerized alpha-olefin is prepared by

   a) contacting an olefin feedstock and at least one oligomerization catalyst to obtain an intermediate product comprising olefin trimers, wherein the olefin feedstock comprises more than 10 wt% nonene, and at least one alpha-olefin selected from the group consisting of decene, dodecene, and tetradecene, and
   b) separating the intermediate product to obtain a poly alpha-olefin.

9. The poly alpha-olefin of claim 8, wherein the poly alpha-olefin has a kinematic viscosity in the range of 1.5 to 2000 cSt at 100 °C.

10. The poly alpha-olefin of claim 8 or 9, wherein the poly alpha-olefin either comprises not more than 20 wt% of oligomers having a carbon count in the range of $C_{18}$ to $C_{20}$ or comprises at least 50 wt% of oligomers having a carbon count of in the range of $C_{27}$ to $C_{40}$.

## Patentansprüche

1. Verfahren für die Oligomerisierung von α-Olefinen, bei dem

   a) ein Ausgangsmaterial von α-Olefinen und mindestens ein Katalysatorsystem zur Oligomerisierung oder Polymerisierung in einem Reaktor unter Bedingungen zur Oligomerisierung oder Polymerisierung in Kontakt gebracht wird, um die α-Olefine zu oligomerisieren oder zu polymerisieren, wobei das Ausgangsmaterial von α-Olefinen mehr als 10 Gew.-% Nonen und mindestens ein α-Olefin ausgewählt aus der Gruppe bestehend aus Decen, Dodecen und Tetradecen enthält,
   b) nicht reagierte α-Olefine entfernt werden, um ein Sumpfprodukt zu erhalten und
   c) gegebenenfalls das Sumpfprodukt hydriert wird, um ein hydriertes Produkt zu erhalten.

2. Verfahren nach Anspruch 1, bei dem das Ausgangsmaterial aus mehr als 10 bis 80 Gew.-% Nonen und mindestens zwei α-Olefinen ausgewählt aus der Gruppe bestehend aus Decen, Dodecen und Tetradecen besteht.

3. Verfahren nach Anspruch 1 oder 2, bei dem das Verfahren den weiteren Schritt der Abtrennung des hydrierten Produkts umfasst, um mindestens zwei Fraktionen von Poly-α-Olefinen mit unterschiedlichen Nennviskositäten zu erhalten.

4. Verfahren nach einem der Ansprüche 1 bis 3, bei dem das Katalysatorsystem einen Katalysator enthält und der Katalysator aus der Gruppe bestehend aus einem Friedel-Crafts-Katalysator, einem geträgertem, reduzierten Metalloxidkatalysator, einer sauren, ionischen Flüssigkeit, einer verbrückten, substituierten, aromatischen Übergangsmetallverbindung und einer unverbrückten, substituierten, aromatischen Übergangsmetallverbindung ausgewählt ist.

5. Verfahren nach einem der Ansprüche 1 bis 4, bei dem das Katalysatorsystem einen Katalysator enthält und der Katalysator aus der Gruppe ausgewählt ist, die aus $AlCl_3$, $AlBr_3$, $BF_3$, einer durch die Formel (1) (Cp-A'-Cp*)$MX_1X_2$ dargestellten Verbindung und einer durch die Formel (2) (CpCp*)$MX_1X_2$ dargestellten Verbindung besteht, wobei M ein Metallzentrum ist, Cp und Cp* gleiche oder unterschiedliche Cyclopentadienylringe sind, die jeweils an M gebunden sind und für Formel (1) mit Null bis Vier Substituenten und für Formel (2) mit Null bis Fünf Substituenten substituiert sind, wobei jede Substituentengruppe unabhängig voneinander eine Restgruppe ist, die eine Kohlenwasserstoff-, substituierte Kohlenwasserstoff-, eine Halogenkohlenwasserstoff-, eine substituierte Halogenkohlenwasserstoff-, eine Carbosilyl- oder Carbogermyl-Gruppe ist, oder Cp und Cp* gleiche oder unterschiedliche Cyclopentadienylringe sind, in denen zwei beliebige benachbarte Substituentengruppen miteinander verbunden sind, um einen substituierten oder unsubstituierten, gesättigten, teilweise ungesättigten oder aromatischen, cyclischen oder polycyclischen Substituenten zu bilden; A' eine Brückengruppe ist und $X_1$ und $X_2$ unabhängig voneinander Hydridreste, Halogenreste, Kohlenwasserstoffreste, substituierte Kohlenwasserstoffreste, Halogenkohlenwasserstoffreste, substituierte Halogenkohlenwasserstoffreste, Carbosilylreste, substituierte Carbosilylreste, Carbogermylreste oder substituierte Carbogermylreste sind oder beide X miteinander verbunden und an das Metallatom gebunden sind, um einen metallacyclischen Ring zu bilden, der 3 bis 20 Kohlenstoffatome enthält.

6. Verfahren nach einem der Ansprüche 1 bis 5, bei dem das Sumpfprodukt ein Poly-α-Olefin mit einer kinematischen Viskosität im Bereich von 1,5 bis 2000 cSt bei 100 °C ist.

7. Verfahren nach einem der Ansprüche 1 bis 6, bei dem das Sumpfprodukt ein Poly-α-Olefin mit mindestens 50 Gew.-% Oligomeren ist, die eine Kohlenstoffzahl im Bereich von $C_{27}$ bis $C_{40}$ haben.

8. Poly-α-Olefin, das aus einem oligomerisierten α-Olefin besteht, wobei das oligomerisierte α-Olefin hergestellt wird, indem

   a) ein Olefin-Ausgangsmaterial und mindestens ein Oligomerisationskatalysator in Kontakt gebracht werden, um ein Zwischenprodukt, das Olefin-Trimere enthält, zu erhalten, wobei das Olefin-Ausgangsmaterial mehr als 10 Gew.-% Nonen und mindestens ein α-Olefin ausgewählt aus der Gruppe bestehend aus Decen, Dodecen und Tetradecen enthält und
   b) das Zwischenprodukt abgetrennt wird, um ein Poly-α-Olefin zu erhalten.

9. Poly-α-Olefin nach Anspruch 8, wobei das Poly-α-Olefin eine kinematische Viskosität im Bereich von 1,5 bis 2000 cSt bei 100 °C aufweist.

**10.** Poly-$\alpha$-Olefin nach Anspruch 8 oder 9, wobei das Poly-$\alpha$-Olefin entweder nicht mehr als 20 Gew.-% Oligomere mit einer Kohlenstoffzahl im Bereich von $C_{18}$ bis $C_{20}$ oder mindestens 50 Gew.-% Oligomere mit einer Kohlenstoffzahl im Bereich von $C_{27}$ bis $C_{40}$ enthält.

## Revendications

**1.** Procédé d'oligomérisation d'alpha-oléfines, le procédé comprenant :

a) la mise en contact d'un stock d'alimentation d'alpha-oléfines et d'au moins un système de catalyseur d'oligomérisation ou de polymérisation dans un réacteur dans des conditions d'oligomérisation ou de polymérisation, afin d'oligomériser ou de polymériser les alpha-oléfines, le stock d'alimentation d'alpha-oléfines comprenant plus de 10% en poids de nonène, et au moins une alpha-oléfine choisie dans le groupe constitué par le décène, le dodécène et le tétradécène ;
b) le retrait des alpha-oléfines non réagies afin d'obtenir un produit de résidus ; et
c) éventuellement l'hydrogénation du produit de résidus afin d'obtenir un produit hydrogéné.

**2.** Procédé selon la revendication 1, dans lequel le stock d'alimentation est constitué de plus de 10 à 80% en poids de nonène, et d'au moins deux alpha-oléfines choisies dans le groupe constitué par le décène, le dodécène et le tétradécène.

**3.** Procédé selon la revendication 1 ou 2, où le procédé comporte une étape supplémentaire consistant à séparer le produit hydrogéné afin d'obtenir au moins deux fractions de poly-alpha-oléfines de viscosités nominales différentes.

**4.** Procédé selon l'une quelconque des revendications 1 à 3, dans lequel le système de catalyseur comprend un catalyseur et le catalyseur est choisi dans le groupe constitué par un catalyseur de Friedel-Crafts, un catalyseur d'oxyde de métal réduit sur support, un liquide ionique acide, un composé de métal de transition aromatique substitué et ponté, et un composé de métal de transition aromatique substitué et non ponté.

**5.** Procédé selon l'une quelconque des revendications 1 à 4, dans lequel le système de catalyseur comprend un catalyseur et le catalyseur est choisi dans le groupe constitué par $AlCl_3$, $AlBr_3$, $BF_3$, un composé représenté par la formule (1) (Cp-A' -Cp*) $MX_1X_2$, et un composé représenté par la formule (2) (CpCp*)$MX_1X_2$ où M est un centre métallique ; Cp et Cp* sont des cycles cyclopentadiényle identiques ou différents qui sont chacun liés à M, et substitués par de zéro à quatre groupements substituants pour la formule (1) et de zéro à cinq substituants pour la formule (2), chaque groupement substituant étant, indépendamment, un groupement radical qui est un hydrocarbyle, hydrocarbyle substitué, halogénocarbyle, halogénocarbyle substitué, silylcarbyle ou germylcarbyle, ou Cp et Cp* sont des cycles cyclopentadiényle identiques ou différents dans lesquels deux groupements substituants adjacents quelconques sont liés afin de former un substituant cyclique ou polycyclique substitué ou non substitué, saturé, partiellement saturé, ou aromatique ; A' est un groupement de pontage ; $X_1$ et $X_2$ sont, indépendamment, des radicaux hydrure, des radicaux halogénure, des radicaux hydrocarbyle, des radicaux hydrocarbyle substitués, des radicaux halogénocarbyle, des radicaux halogénocarbyle substitués, des radicaux silylcarbyle, des radicaux silylcarbyle substitués, des radicaux germylcarbyle, ou des radicaux germylcarbyle substitués ; ou les deux X sont reliés et fixés à l'atome de métal afin de former un cycle de métallacycle contenant de 3 à 20 atomes de carbone.

**6.** Procédé selon l'une quelconque des revendications 1 à 5, dans lequel le produit de résidus est une poly-alpha-oléfine ayant une viscosité cinématique dans la plage allant de 1,5 à 2000 cSt à 100°C.

**7.** Procédé selon l'une quelconque des revendications 1 à 6, dans lequel le produit de résidus est une poly-alpha-oléfine comprenant au moins 50% en poids d'oligomères ayant un nombre de carbones dans la plage allant de $C_{27}$ à $C_{40}$.

**8.** Poly-alpha-oléfine constituée d'une alpha-oléfine oligomérisée, où ladite alpha-oléfine oligomérisée est préparée par

a) la mise en contact d'un stock d'alimentation d'oléfines et d'au moins un catalyseur d'oligomérisation afin d'obtenir un produit intermédiaire comprenant des trimères d'oléfine, où le stock d'alimentation d'oléfines comprend plus de 10% en poids de nonène, et au moins une alpha-oléfine choisie dans le groupe constitué par le décène, le dodécène et le tétradécène, et
b) la séparation du produit intermédiaire afin d'obtenir une poly-alpha-oléfine.

**9.** Poly-alpha-oléfine selon la revendication 8, où la poly-alpha-oléfine possède une viscosité cinématique dans la plage allant de 1,5 à 2000 cSt à 100°C.

**10.** Poly-alpha-oléfine selon la revendication 8 ou 9, où la poly-alpha-oléfine comprend au plus 20% en poids d'oligomères ayant un nombre de carbones dans la plage allant de $C_{18}$ à $C_{20}$, ou bien comprend au moins 50% en poids d'oligomères ayant un nombre de carbones dans la plage allant de $C_{27}$ à $C_{40}$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003020856 A
- WO 2007011973 A
- US 6355603 B
- US 5264203 A
- US 20070043248 A
- US 2009005279 A